# EUROPEAN PATENT SPECIFICATION

(11) **EP 3 085 306 B1**
(45) Date of publication and mention of the grant of the patent: **01.11.2017**
(21) Application number: 15164576.9
(22) Date of filing: 22.04.2015
(51) Int. Cl.: A61B 5/15, A61B 90/00, A61B 90/90

(54) **MEDICAL PRODUCT AND METHOD FOR AUTHENTICATING THE SAME**
MEDIZINISCHES PRODUKT UND VERFAHREN ZUR AUTHENTIFIZIERUNG DESSELBEN
PRODUIT MÉDICAL ET PROCÉDÉ POUR AUTHENTIFIER CELUI-CI

(43) Date of publication of application: 26.10.2016
(73) Proprietor: Roche Diabetes Care GmbH, 68305 Mannheim (DE); F. Hoffmann-La Roche AG, 4070 Basel (CH)
(72) Inventor: Ebert, Sven, 8006 Zürich (CH); Thös, Bruno, 67657 Kaiserslautern (DE); Rehme, Frank, 64683 Einhausen (DE)
(74) Representative: Herzog, Fiesser & Partner Patentanwälte PartG mbB

(56) References cited:
- EP-A2- 2 492 879
- WO-A1-00/65541
- US-A1- 2002 187 347
- US-A1- 2003 102 661
- US-A1- 2009 136 693
- US-A1- 2013 302 591

## Description

### Field of the invention

The invention relates to a medical product which is to be authenticated, an authentication device and an authentication system, and to an authentication method for testing authenticity of an authenticable medical product. The present invention specifically may be used in the field of anti-counterfeiting of medical aids such as medical devices, test elements, packagings of medical products or pharmaceuticals. Still, other fields of application are feasible and will be discussed in further detail below.

### Related art

Manufacturers, distributors, vendors and users of various types of products increasingly are faced with the problem of product piracy and product counterfeiting. Specifically, in the field of pharmaceutical manufacturing and medical technology, counterfeiting imposes significant technical challenges, since falsified products may lead to severe damages, injuries and long-term health problems. Consequently, adequate authentication strategies have to be implemented.

Various technologies for product authenticating are generally known in the art, including methods and technologies applicable in pharmaceutical manufacturing and medical technology. As an example, visible markings may be applied to vessels and packaging. Further, it is generally known that chemical fingerprints of products and packagings may be modified and may be used for authentication. Additionally, electronic identifiers may be used, such as electronic identification chips, or optical identifiers, such as barcodes and/or holograms. Further, technologies providing an "artificial DNA" are generally known.

For detecting various types of identifiers for authentication purposes, the use of x-ray imaging, such as using computer tomography, are generally known in the art. This is mainly due to the fact that, specifically in the field of computer tomography, the overall complexity and cost of computer tomography systems have decreased. Miniaturized computer tomography systems are available and may be used for authentication purposes.

US 2013/0302591 A1 discloses a composite film having barrier properties for use as in-mold labels as well as articles having such in-mold labels. Thereby, a product identifier may directly be incorporated into a product packaging. One general disadvantage of this type of incorporating product identifiers resides in the fact that the label is generally visible from the outside and may easily be detected. Therefore, such in-mold labels generally are disadvantageous as a protection against counterfeiting.

In US 2003/0102661 A1, an authenticable three-dimensional object and a method for protecting and identifying objects is disclosed. The object specifically may be an article made by a foundry, casting or a similar technique. Therein, marker elements are included in the main material of the article during manufacture thereof, the nature of the marker elements being different from that of the main material of the article, so as to react to means of nondestructive investigation of the internal structure of the article. Specifically, after the article has been made, the article is identified by identifying marker elements in the internal structure of the article using x-ray tomographic analysis technique. Therein, a position of the marker elements is determined by using a reference plane and at least one section plane parallel to the reference plane. Graphics documents are produced, including pictures taken via tomographic x-ray examination of the article.

Although the method disclosed in US 2003/0102661 A1 provides a significant protection against counterfeiting, the method disclosed therein is rather complex. Thus, the application of this method is generally limited to small batch series rather than to high volumes or mass production.

In US 6,584,214 B1, three-dimensional characteristics of a complex physical structure are used to generate a unique identifier. In effect, the characteristics represent the basis of a physical one-way hash function that facilitates ready derivation of an identifier based on the physical structure, the structure itself being very difficult to reproduce given only the identifier. The characteristics may be read using a non-contact probe and without the need for precise registration.

Thus, generally, known prior art documents describe embedding homogeneous authentication bodies in a matrix material, wherein information is encoded through a spatial distribution of the homogeneous authentication bodies.

Known methods for authentication imply several technical shortcomings or challenges. Firstly, a disadvantage of known authentication methods resides in the fact that the means for authentication may easily be detected. As an example, a doping of a matrix material may simply be detected by using infrared or ultraviolet light. Magnetic identifiers or electronic identification chips must be accessible electrically and/or via a wireless access and, thus, may simply be detected by using a reader. Artificial DNA generally uses fluorescence markers, in order to characterize and identify the location of the artificial DNA. Consequently, due to the simple detectability of the authentication method, counterfeiting of identifiers is enabled, since identifiers may easily be detected and copied.

Further, several identifiers require a destructive method of identification. As an example, artificial DNA requires a destructive sampling of the adhesive containing the artificial DNA. Further, the adhesive has to be stable and transparent over the product lifetime, and an optical access to the identifier has to be provided. Consequently, generally, identifiers and authentication methods are preferred which are invisible and widely undetectable and which do not require a destructive reading.

Further, known identification methods using x-ray tomography, such as disclosed in US 2003/0102661 A1 and/or US 6,584,214 B1, generally require a complex pattern of evaluation of randomly distributed particles within the article, including the necessity of providing enormous computational resources for storing and comparing individual images of particle distribution. Therefore, additionally, an authentication method is generally desired which is simple to implement and which, still, provides a safe and reproducible way of authenticating products such as medical consumables and/or pharmaceuticals.

In other fields of technology, several techniques for manufacturing pigments and microparticles are known. Thus, as an example, DE 41 05 319 A1 discloses coated micro-beads made of glass, containing a coating of a metal compound having a refractive index of more than 2. The micro-beads are distributed within a binder polymer and provide a colored outer surface for decorative purposes.

Similarly, US 7,498,081 B2 discloses aqueous chemical routes and finished compositions of a core-shell particulate material for application in crystallizable glass enamel. Further, a composition of particles containing a shell of inorganic oxides or mixed-metal inorganic oxides and a core material of complex inorganic colored pigment, wherein the shell material is comprised of any single oxide or combination of oxides is disclosed. The resulting particles function in glass enamel formulations as coloring agents and additives for partial crystallization of the glass material, and provide modification of glass melt temperature, durability, acid resistance, and other desirable properties.

### Problem to be solved

It is therefore an objective of the present invention to provide an authenticable medical product, an authentication device and an authentication system as well as a method for testing authenticity of an authenticable medical product which at least partially overcome the above-mentioned shortcomings and challenges of known devices and methods. Specifically, the invention shall provide means and devices for authenticating medical products in a simple, efficient and reliable way.

### Summary of the invention

This problem is solved by an authenticable medical product, an authentication device, an authentication system and a method for testing authenticity of an authenticable medical product, with the features of the independent claims. Preferred embodiments, which might be realized in an isolated fashion or in any arbitrary and feasible combination are listed in the dependent claims.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect of the present invention, an authenticable medical product is disclosed, comprising a matrix material with at least one authentication body embedded into the matrix material. The authentication body is heterogeneous and comprises a core material forming a core of the heterogeneous authentication body, and at least one cover layer at least partially covering the core of the heterogeneous authentication body. At least one material of the at least one cover layer is different from the core material and the matrix material.

As used herein, the expression "medical product" generally refers to an arbitrary item or article which may be used in the field of medical diagnostics, surgery, therapy or any other field of medicine, medical technology, diagnostics, treatment or therapy. Thus, as will be outlined in further detail below, the medical product may specifically be or may comprise at least one pharmaceutical item and/or a packaging thereof. Additionally or alternatively, the medical product may be or may comprise at least one medical and/or therapeutic aid, such as at least one puncture aid and/or at least one medication aid, such as at least one infusion kit. Additionally or alternatively, the medical product may comprise one or more medical consumable products, such as one or more test elements, more specifically test strips or other test elements for detecting at least one property of a sample, such as for quantitatively and/or qualitatively detecting at least one analyte in a sample of a body fluid. Additionally or alternatively, the medical product may comprise one or more sensors or sensor elements, specifically insertable sensors which are configured to be inserted into a body tissue of a user, such as for monitoring a concentration of one or more analytes in the body tissue or in a body fluid over a period of time, specifically for continuous monitoring. Additionally or alternatively, the medical product may comprise one or more of a meter housing; an electronic circuit board; a sensor for continuous subcuteanous monitoring; a circuit board for an electronic medical device; a carrier with a plurality of test pads. Other examples will be given in further detail below.

As further used herein, the term "authenticable" generally refers to the property of the medical product being capable of being authenticated. Thus, generally, the term "authenticate" refers to the process of determining the authenticity of the product, i.e. of determining the origin of the medical product and/or the process of verifying the fact that the medical product actually originates from a nominal manufacturer, distributor or vendor. Thus, generally, the term "authenticable medical product" refers to a medical product which may be authenticated.

As further used herein, the term "matrix material" refers to a material which may form a body of the medical product and/or of a part thereof. The matrix materials specifically may be subject to a molding and/or shape forming process, such as by molding, casting, die casting or any other shape forming process. As an example, the matrix material, as will be outlined in further detail below, may be or may comprise a plastic material, such as a thermoplastic, a duroplastic or elastomeric material, which may be shaped and which may define the shape of the medical product and/or of a part thereof.

As further used herein, the term "authentication body" refers to an item, article or object which may be detected and/or of which at least one property may be detected by a detection and, thus, which may form the basis of an authentication process. Specifically, as outlined in further detail below, the at least one authentication body may comprise one or more authentication bodies such as particles.

The authentication body is heterogeneous, which, generally, refers to the fact that the authentication body comprises at least two distinguishable components, i.e. at least the above-mentioned core and at least the above-mentioned at least one cover layer. The term "distinguishable" generally refers to the fact that these components of the authentication body may be distinguished by using at least one detection step, such as by using at least one imaging step, more preferably a tomographic imaging step and, still more preferably, at least one x-ray tomographic step.

As outlined above, the at least one cover layer at least partially covers the core of the authentication body. Thus, the at least one cover layer specifically may form a shell which fully or partially encloses the core. The at least one cover layer may comprise one or more cover layers. The material of the at least one cover layer, wherein one material or more than one material may be used, is different from the core material and the matrix material. In this regard, the term "different" generally refers to the fact that the material of the at least one cover layer may be distinguished both from the core material and the matrix material in a detection step, such as by using one or more of the above-mentioned detection methods, which will be outlined in further detail below. As an example, the at least one cover layer may be distinguished from the core material and the matrix material by its x-ray contrast and/or by having a different x-ray density than the core material and the matrix material.

The material of the at least one cover layer preferably differs from the core material in such a way that the at least one cover layer is distinguishable from the core by using at least one detection method or detection technique. Preferably, the detection method is a nondestructive imaging method, such as an x-ray imaging method. As an example, the nondestructive imaging method may comprise a tomographic imaging method, such as a computer tomography (CT) and/or an x-ray tomography. Still, additionally or alternatively, other imaging methods may be used for distinguishing the cover layer from the core. Generally, preferably, the matrix material, the core and the cover layer mutually are distinguishable by using the detection method.

As an example of distinguishing the matrix material, the core and the cover layer, a distinguishing by x-ray imaging is preferred. As used herein, x-ray generally refers to electromagnetic radiation having a wavelength of 0.25 nm, such as a wavelength of 0.25 nm to 1 pm, and/or an energy of 1 keV or more, such as 1 keV to 250 keV. In order to be distinguishable, the matrix material, the core and the cover layer, as an example, may provide a differing absorption coefficient in at least one frequency range and/or wavelength range of the x-ray spectrum, such as in a range of 0.25 nm to 5 pm or a partial range thereof. As an example, differing absorption coefficients and/or differing x-ray densities may be used, as known in the field of crystallography. Thus, as an example, the material of the at least one cover layer may differ from the core material by at least one property rendering the core and the cover layer distinguishable by x-ray imaging, preferably at least one property selected from the group consisting of an x-ray density or an x-ray absorption coefficient. Similarly, the material of the at least one cover layer may differ from the matrix material by at least one property rendering the matrix material and the cover layer distinguishable by x-ray imaging, preferably at least one property selected from the group consisting of an x-ray density or an x-ray absorption coefficient. Again, additionally or alternatively, the core material may differ from the matrix material by at least one property rendering the core and the matrix material distinguishable by x-ray imaging, preferably at least one property selected from the group consisting of an x-ray density or an x-ray absorption coefficient.

X-ray densities or x-ray absorption coefficients are generally well-known in the art or may simply be determined by experiment, such as by measuring an x-ray absorption at various thicknesses of a specific material. Further, x-ray absorption coefficients and/or x-ray densities are widely known in the field of material sciences and/or medical imaging. Thus, generally, the core, the cover layer and the matrix material may be distinguishable by x-ray imaging, such as by computer tomography.

The authenticable medical product, as outlined above, comprises at least one authentication body embedded into the matrix material. As used herein, the term "embedding" generally may refer to the fact that the authentication body is fully or at least partially surrounded by the matrix material. As an example, the authentication body may be cast into the matrix material by some type of casting or molding process, in order to fully embed the authentication body into the matrix material. Additionally or alternatively, the authentication body may be embedded in between two or more layers of the matrix material.

One or more than one authentication bodies may be used and may be provided in the authenticable medical product. In case a plurality of authentication bodies is provided in the matrix material, as an example, the authentication bodies may be provided in the form of a powder and/or particles, each particle having at least one core and at least one cover layer. The authentication bodies specifically may have a diameter or equivalent diameter of 0.1 µm to 500 µm, such as 1 µm to 100 µm. However, other ranges are generally feasible. The range of 0.1 µm to 500 µm, such as 1 µm to 100 µm, however, has turned out to advantageous, specifically in the field of manufacturing the authenticable medical product, such as by casting and/or molding such as injection molding. The authenticable medical product, as an example, may be manufactured by injection molding, thereby injecting a mixture of the matrix material and the at least one authentication body into a mold. As an example, reference may be made to the manufacturing methods disclosed in one or more of documents DE 41 05 319 A1 or US 7,498,081 B2 disclosed above. Additionally or alternatively, other ways of manufacturing may be used.

The matrix material specifically may be selected from the group consisting of: a plastic material; a glass material; a ceramic material; a metal. Still, other materials are feasible. The matrix material specifically may comprise or may be a homogeneous matrix material. Therein, the matrix material, according to the requirements of the authenticable medical product, may be opaque and/or may fully or partially be transparent. In case a transparent matrix material is used, preferably, the at least one authentication body also is transparent, having the same or at least substantially the same absorption properties in the visible spectral range.

The matrix material may comprise or may be a single matrix material or may comprise a plurality of matrix materials. The matrix material, as an example, may be a curable matrix material, which may, as an example, be cast and/or injected into a mold in a liquid or pasty format and which may be cured and/or hardened inside the mold. Generally, the matrix material, in the final form of the authenticable medical product, specifically may be hardened and/or a hard material. Still, for some medical products, also elastic and/or deformable matrix materials may be used.

As outlined above, in one embodiment, a plurality of matrix material layers may be provided, and the at least one authentication body may be embodied in between at least two of the matrix material layers. Thus, as an example, the authenticable medical product may also comprise a layer setup, having the at least one authentication body embedded in between two matrix material layers. Still, preferably, the at least one authentication body is embedded in a homogeneous amount of the matrix material, without having a layer setup.

The at least one authentication body, as outlined above, specifically may comprise a plurality of particles. Generally, the one or more authentication bodies specifically may be selected from the group consisting of: a sphere, a fiber, an amorphous particle, a lamella, a bead. Still, other geometric shapes are feasible. The core material specifically may be selected from the group consisting of: a plastic material, a ceramic material, a metal, a metal alloy, a glass, a quartz, a crystalline material, a silicone. Still, other materials and/or mixtures of materials are feasible.

The authentication body may have a single cover layer or may comprise a multilayer setup. Thus, in case of a multilayer setup, the multilayer setup may comprise a multiplicity of cover layers, having one and the same cover layer thickness or having differing cover layer thicknesses.

The product specifically may comprise at least one additional disguise material embedded into the matrix material, wherein the disguise material may lower a detectability of the authentication body. Thus, as an example, the disguise material may comprise a powder. The disguise material, as an example, may have an x-ray absorption coefficient and/or an x-ray density in between the respective absorption coefficients and/or x-ray densities of the matrix material on the one hand and the core and cover layer on the other hand, in order to lower the contrast of the authentication body. As an example, the disguise material may comprise barium sulfate.

Specifically, the authentication body may be invisible from the outside of the authenticable medical product under inspection by the naked eye. Thus, as will be outlined in further detail below, the authentication body may have the same or a similar color as the matrix material of the authenticable medical product. In case the matrix material is fully or partially transparent, the authentication body, i.e. the core material and/or the material of the at least one cover layer, specifically may have the same or a similar index of refraction in the visible spectral range.

The authenticable medical product specifically may be selected from the group consisting of: a puncture aid for generating at least one opening in a skin of a user, a lancet, a syringe, an infusion device for administering a liquid to a body tissue (such as an insulin infusion kit), a plaster, a packaging for a medical device or product, a pharmaceutical (such as a pill, a bolus, a tablet), a test element for detecting at least one property of a sample, a test strip. Still, other authenticable medical products may be embodied according to the present invention. Further, only parts of the named products and/or other medical products may be embodied according to the invention.

In a further aspect of the present invention, an authentication device for authentication of at least one authentical medical product according to the invention, such as according to any one of the above-mentioned embodiments and/or according to one or more of the embodiments disclosed in further detail below, is disclosed. The authentication device comprises at least one detector for detecting the at least one authentication body and for distinguishing the core and the cover layer of the authentication body. The authentication device further comprises at least one evaluation device, wherein the evaluation device is adapted for decoding the at least one authentication body.

For definitions of the elements of the authentication device, reference may be made to the authenticable medical product as outlined above. A detector, as used herein, generally refers to a device adapted for performing one or more detection methods, such as one or more of the detection methods outlined above. As an example, the at least one detector may comprise at least one imaging device, more preferably at least one x-ray imaging device and/or at least one tomographic imaging device, such as an x-ray tomographic imaging device and/or a computer tomographic device.

As further used herein, the term "evaluation device" generally refers to an arbitrary device adapted for decoding the at least one authentication body. Therefore, the evaluation device may be adapted to evaluate one or more items of information provided by the at least one detector, such as image data provided by the at least one detector. The term "evaluate" generally refers to the process of deriving at least one item of information from data, such as data and/or information provided by the detector. The term "decoding" generally refers to the process of deriving at least one authentication information from the data, i.e. at least one item of information which relates to the above-identified authentication process. Thus, as an example, the decoding may imply deriving at least one result, such as a result relating to the authenticity of the authenticable medical product, such as information regarding a manufacturer, vendor or provider and/or other items of information. As will be outlined in further detail below, the authentication body may further be used for storing items of information, such as a batch number or lot number and/or calibration information for using the authenticable medical product. Examples of decoding and/or deriving authentication information and/or additional information will be given in further detail below, in the context of the authentication method disclosed in further detail below.

As outlined above, the detector specifically may comprise at least one tomographic imaging device. Further, the at least one evaluation device specifically may comprise at least one processor, including microprocessors and/or application-specific integrated circuits (ASIC). The evaluation device specifically may be adapted to generate at least one item of information regarding an authenticity of the authenticable medical product.

In a further aspect of the present invention, an authentication system is disclosed. The authentication system comprises at least one authentication device according to any one of the embodiments disclosed above and/or as disclosed in further detail below. The authentication system further comprises at least one authenticable medical product according to the present invention, such as according to any one of the preceding embodiments and/or according to any one of the embodiments disclosed in further detail below.

In a further aspect of the present invention, an authentication method for testing authenticity of the authenticable medical product according to the present invention is disclosed. The authentication method comprises detecting and decoding the at least one authentication body embedded into the authenticable medical product. As outlined above, the detecting of the at least one authentication body specifically may comprise using at least one nondestructive imaging method. The nondestructive imaging method specifically may be or may comprise a tomographic imaging method, such as computer tomography and/or x-ray tomography. Other imaging methods may be used additionally and/or alternatively.

The detecting of the at least one authentication body may comprise generating at least one three-dimensional image of at least a part of the authenticable medical product. The detecting of the at least one authentication body specifically may imply and/or comprise detecting one or more of: a contrast between the matrix material and the core material; a contrast between the matrix material and the material of the at least one cover layer; a contrast between the core material and the material of the at least one cover layer.

The decoding specifically may comprise deriving at least one measurement value from the detecting of the at least one authentication body. The at least one measurement value may be used for encoding the at least one authentication information from the authentication body. Thus, by choosing an appropriate geometry of the at least one authentication body, such as an appropriate thickness and/or an appropriate geometric shape, information may be encoded into the authentication body. Thus, as an example, a layer thickness of the cover layer may be used for encoding information, such as by dividing possible range of the layer thicknesses into sub-ranges and assigning information to each range of layer thickness. Additionally or alternatively, the authentication method may simply comprise comparing the layer thickness of the cover layer with a predetermined thickness or thickness range, wherein a positive result of the comparison may lead to the authentication information indicating that the product is authentic. The at least one measurement value may, thus, be compared with at least one nominal value or nominal value range during the decoding step of the authentication method.

The measurement value specifically may comprise one or more of: a diameter or equivalent diameter of the authentication body; a thickness of the cover layer; a mean diameter of a plurality of authentication bodies; a mean thickness of cover layers; a spatial distribution of authentication bodies; a geometric shape of the authentication body.

The decoding of the authentication method may further comprise generating at least one authentication result, also referred to as at least one item of authentication information. The authentication result specifically may comprise at least one item of information regarding an authenticity of the authenticable medical product, such as an item of information regarding the fact that the authenticable medical product is authentic or not.

Besides the at least one authentication result, the decoding may further comprise generating at least one additional item of information encoded in the authenticable medical product, specifically encoded in the at least one authentication body. Thus, as outlined above, besides at least one item of information regarding the authenticity of the authenticable medical product, additional information may be encoded by using the at least one authentication body. Thus, as an example, the at least one additional item of information may comprise at least one item of information characterizing one or more of: an identity of the authenticable medical product; a manufacturer of the authenticable medical product; a vendor of the authenticable medical product; a distributor of the authenticable medical product; a property of the authenticable medical product, such as an expiry date, a content, a quality grade or other properties of the authenticable medical product; an intended use of the authenticable medical product; a calibration of the authenticable medical product and/or a device interacting with the authenticable medical product; a batch number of the authenticable medical product. Other items of information may additionally or alternatively be encoded.

The invention further discloses and proposes a computer program including computer-executable instructions for performing the authentication method according to the present invention in one or more of the embodiments enclosed herein when the program is executed on a computer or computer network. Specifically, the computer program may be stored on a computer-readable data carrier. Thus, specifically, one, more than one or even all of the method steps as indicated above may be performed by using a computer or a computer network, preferably by using a computer program. Specifically, the method steps of detecting and/or decoding may fully or partially be computer-implemented, such as by controlling the detection via computer and/or using a computer algorithm for decoding.

The invention further discloses and proposes a computer program product having program code means, in order to perform the method according to the present invention in one or more of the embodiments enclosed herein when the program is executed on a computer or computer network. Specifically, the program code means may be stored on a computer-readable data carrier.

Further, the invention discloses and proposes a data carrier having a data structure stored thereon, which, after loading into a computer or computer network, such as into a working memory or main memory of the computer or computer network, may execute the method according to one or more of the embodiments disclosed herein.

The invention further proposes and discloses a computer program product with program code means stored on a machine-readable carrier, in order to perform the method according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier. Specifically, the computer program product may be distributed over a data network.

Finally, the invention proposes and discloses a modulated data signal which contains instructions readable by a computer system or computer network, for performing the method according to one or more of the embodiments disclosed herein.

Preferably, referring to the computer-implemented aspects of the invention, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

Specifically, the present invention further discloses:
- A computer or computer network comprising at least one processor, wherein the processor is adapted to perform the method according to one of the embodiments described in this description,
- a computer loadable data structure that is adapted to perform the method according to one of the embodiments described in this description while the data structure is being executed on a computer,
- a computer program, wherein the computer program is adapted to perform the method according to one of the embodiments described in this description while the program is being executed on a computer,
- a computer program comprising program means for performing the method according to one of the embodiments described in this description while the computer program is being executed on a computer or on a computer network,
- a computer program comprising program means according to the preceding embodiment, wherein the program means are stored on a storage medium readable to a computer,
- a storage medium, wherein a data structure is stored on the storage medium and wherein the data structure is adapted to perform the method according to one of the embodiments described in this description after having been loaded into a main and/or working storage of a computer or of a computer network, and
- a computer program product having program code means, wherein the program code means can be stored or are stored on a storage medium, for performing the method according to one of the embodiments described in this description, if the program code means are executed on a computer or on a computer network.

The methods and devices as disclosed by the present invention provide a large number of advantages over known methods and devices in the field of product authentication. Thus, generally, the authentication may be performed in a confined regional section of the product, rather than using the overall authenticable medical product, since the authentication body may spatially be confined to a section of the authenticable medical product. The authentication body, as opposed to e.g. artificial DNA, is located inside the matrix material, embedded into the matrix material.

By using computer tomography, a relative x-ray density distribution of the authenticable medical product and/or a part thereof may be performed. By performing a computer tomographic analysis, the presence of the authentication body may be detected, and, additionally, the authentication information may be derived thereof, such as from the thickness of the core and/or the cover layer. The authentication may be performed in a contactless fashion and in a nondestructive fashion. Further, the authentication may be rendered independent from the optical properties of the authentication body and/or the authenticable medical product. Consequently, optically invisible or hardly visible authentication bodies may be used.

The authentication body may be embodied small compared to the dimensions of the authenticable medical product. As outlined above, preferred ranges of diameter or equivalent diameter are 0.1 µm to 500 µm, such as 1 µm to 100 µm. A relative x-ray density of the authentication body, i.e. the core and/or the cover layer, may significantly differ from the matrix material of the product, thereby rendering the authentication body and its properties visible and detectable to the detection method, such as to computer tomography. As an example, the x-ray density of the core may differ from the x-ray density of the matrix material by at least 10 %. Similarly, the x-ray density of the cover layer may differ from the x-ray density of the matrix material by at least 10 %. Preferably, the x-ray densities of the core material and the cover layer material also differ, such as also by 10 %. An authentication of the authentication body and, thus, an authentication of the product may be performed by using the geometry of the authentication body and/or material properties and/or a frequency, a relative frequency, a number or an occurrence of at least one authentication body. As an example, a volume content of the authentication body, of the core and/or of the at least one cover layer may be used for deriving the at least one item of authentication information.

Authentication bodies, generally, may be embodied as robust and/or sturdy bodies, thereby allowing for mixing these authentication bodies into the matrix material and rendering the authentication bodies robust against shaping processes, such as injection molding or other manufacturing processes, without destroying the properties of the authentication bodies.

The one or more authentication bodies may be brought into the authenticable medical product and/or into the matrix material in various ways. The method of introduction generally may be adapted to the matrix material and/or to the product. Thus, as an example, in case the matrix material comprises a plastic material, the at least one authentication body may be mixed into the matrix material, as is generally done with filling materials in molding technology or inkjet technology. In case of thermoplastic material, the at least one authentication body may be mixed into a master batch. In case the product and/or the matrix material fully or partially consist of one or more of glass, ceramics or metal, the at least one authentication body, as an example, may be introduced into a melt. In case of deformable metals and/or plastic materials, the at least one authentication body may also be brought in between two or more layers or foils of the matrix material, followed e.g. by a pressing step. In case a powder process is used, such as in case of powder injection molding, the at least one authentication body, as an example, may be mixed into the powder. Composite materials may be treated similar to the matrix material. In case layered materials are used, such as composites of glass and/or carbon fibers, wooden laminates or foils, the one or more authentication bodies may be brought in between the layers, such as during a lamination process. Products manufactured by microsystem technology may also make use of one or more of the above-mentioned techniques, in order to adapt the processing to the microsystem technology. Further, it may also be possible to implement one or more processing steps for manufacturing the at least one authentication body in situ, as a part of the general processing step. Thus, the forming of one or more authentication bodies having the core and the at least one layer may be part of a general manufacturing process of the authenticable medical product. In case techniques such as 3D printing, additive manufacturing or rapid prototyping, the at least one authentication body may also be introduced into the product during manufacturing.

The at least one authentication body may be kept at small dimensions, in order to render them detectable by micro detection techniques, only, such as micro computer tomography (micro-CT) and/or nano computer tomography (nano-CT). These devices generally comprise one or more micro or nano focus x-ray tubes and are generally adapted to analyze products at a very high resolution. As an example, reference may be made to the nanotomographic devices and methods disclosed in E. L. Principe: "Practical Nanotomography - Focused ion beam electron microscopy is key for advanced nanotomography", Photonics Spectra, Features, May 2008 (available online via http://www.photonics.com/Article.aspx?AID=33531).

The at least one authentication body, as an example, may comprise one or more spheres, such as one or more glass spheres, having a core and at least one layer. The spheres, as an example, may be characterized by their diameter or equivalent diameter, by their diameter distribution or scattering, by their density or their density distribution or density scattering. These parameters may be used to encode one or more items of authentication information. Further, spheres belonging to different diameter groups or density groups may be used. By choosing an appropriate diameter and/or density, and/or by choosing an appropriate ratio of spheres having a first density and spheres having a second density, encodings such as a date and/or a batch number may be introduced.

Further, the at least one authentication body may comprise one or more metal plates, which also are referred to as flakes. These metal plates also may comprise a core and one or more layers. These flakes generally may be manufactured by using microsystem technology, at low cost. A patterning may be performed, e.g. using photolithography. Further, geometries of the flakes may be produced, such as rectangles and/or circles, and/or other geometries. Flakes, as an example, may be manufactured from metallic and/or thermoplastic materials.

Further, authentication bodies may be embodied as one or more flakes comprising a plurality of cover layers of different x-ray density. As an example, a layer thickness, layer thickness ratios and/or density ratios, and/or a geometry, such as a patterning, of the flakes may be used for identification and/or authentication. The x-ray density of the layers in the flakes may be adjusted by intermixing additional layer materials, such as during evaporation.

Flakes may easily be manufactured by coating and/or patterning on a plastic foil, which may be part of the product. The foil, as an example, may be combined with other foils by lamination and/or may be introduced into the product.

In case coated spheres are used or also in case of other authentication bodies, a layer thickness and/or an x-ray density of the one or more layers may be used as authentication information and/or for encoding authentication information and/or additional information. The one or more layers, as an example, may be manufactured by evaporation, galvanic coating and/or coating by precipitation. Other coating techniques may be used.

In case transparent or semitransparent materials or matrix materials are used, the authentication body, i.e. the core material and/or the cover layer material, may also be transparent. As an example, the core and/or the one or more layers may be manufactured by using glass and/or a transparent plastic material, preferably having an optical index of refraction which is widely adapted or assimilated to the matrix material, such as having an index of refraction which does not deviate from an index of refraction of the matrix material by more than 20%, preferably by no more than 10%. In case the matrix material comprises, as an example, one or more transparent thermoplastic materials, such as PMME and/or polycarbonate, the authentication body, as an example, may be manufactured from one or more duromeric materials, such as an epoxy resin, and/or one or more elastomeric materials, such as silicone and/or silicone rubber. The use of silicone and/or silicone rubber generally provides the advantage that the index of refraction is well adapted to be distinguishable in x-ray tomography, due to the content of silicon and oxygen. A high x-ray contrast between the authentication body comprising the silicone or silicone rubber on the one hand side and the thermoplastic matrix material is given. In case glass is used as a matrix material, high melting point glass components, such as quartz glass, may be used as the authentication body and/or as a part thereof. In case a translucent or colored glass is used, as an example, authentication bodies may be used containing a kovar component, wherein kovar is a high melting point alloy having, as an example, 54 mass percent iron, 29 mass percent nickel and 17 mass percent cobalt. Kovar also reduces thermal tensions in the glass.

In case the at least one authentication body is optically visible from the outside of the authenticable medical product, the actual function of the authentication body may be disguised by appropriate means. Thus, as an example, the authentication body may be colored, in order to provide a coloring of the product, such as a metallic effect, and/or for other decorative purposes. In this case, as an example, the authentication function may be introduced into a multi-layer foil, in order to render the outer surface of the authentication body inconspicuous.

The disguise of the at least authentication body, as outlined above, may also be improved by an additional disguise material, such as one or more filler materials having a high x-ray density, such as barium sulfate. Thereby, the general transmission of x-rays may be reduced, and the authentication process must be performed at a relatively high x-ray energy.

Further, the detection of the at least one authentication body may be reduced by lowering the content and/or the proportion of the authentication body or authentication bodies, as compared to the overall volume of the product. In order to find the remaining authentication bodies, specifically in thin-wall products, the visualization of the at least one authentication body may be performed by thermal measurements, such as by measuring a thermal answer of the product during heating and/or cooling, such as by using a flashlight and/or an infrared camera, specifically in case the thermal behavior of the at least one authentication body differs from the matrix material. Therein, typically, in infrared images, the authentication body is visualized as defect in the infrared image, thereby allowing to identify the location of the authentication body. Thus, generally, the detection of the at least one authentication body may comprise at least one step of identifying a location of the at least one authentication body, such as before performing an imaging analysis or detection by at least one imaging method, such as a computer tomography. In this case, the imaging, specifically the tomographic imaging, may be narrowed to the actual region of interest within the product. Additionally or alternatively, in order to find the at least one authentication body, other types of detectors may be used, such as a metal detector, specifically in case the authentication body comprises one or more magnetic materials such as nickel.

The core material and/or the layer material may also be embodied as one or more biocompatible materials. As an example, an outer layer of the at least one authentication body may comprise a biocompatible layer. Additionally or alternatively, inert materials, such as gold, may be used. These types of small authentication bodies may even be introduced into food or edibles and/or into pharmaceuticals suited for oral consumption.

The detecting and/or the decoding of the at least one authentication body in the method according to the present invention may also comprise using at least one model of the at least one authentication body and/or of the authenticable medical product including the at least one authentication body. As an example, one or more CAD models of the authenticable medical product and/or the authentication body may be stored and/or used by at least one evaluation device, such as a processor. By comparing a multiplicity of authentication bodies from a product, a statistical distribution of a plurality of authentication bodies may be generated, such as histograms indicating geometry distributions and/or relative x-ray density. Possible parameters which characterize the statistical distribution of the measured parameters are, e.g., the so-called process capability indices (PCI, cₚₖ) which may also be used, for characterizing the distributions of e.g. the layer thickness and/or the diameter of the authentication bodies within the authenticable medical product. Other parameters may also be used. By comparison of the cₚₖ values with known distributions of the authentication bodies used in the relevant batches, the authenticity of the authenticable medical product may be evaluated.

As outlined above, the method of authentication as proposed in the present invention may be invisible from the outside of the product and, thus, may remain concealed to potential counterfeiters. Since the authentication bodies may be kept rather small, they may only be visible with state of the art computer tomography and, otherwise, may not be distinguished from regular defects or unwanted contaminations.

The authentication bodies may be manufactured on a large scale, such as by using microsystem technology. For introducing the authentication bodies into a master batch of the matrix material, the overall process for shaping the matrix material or for generating a packaging, generally, does not have to be modified to a large extent. Authentication bodies, thus, may be implemented into products in use.

Further, the detection of the authentication bodies may be performed in a contactless and nondestructive way and is reversible, as opposed e.g. to common technologies for detecting artificial DNA. Further, recent progress in computer tomography, such as miniaturization and increasingly powerful processes generally lower the costs for the detection process. CT devices are generally also available as desktop devices and may be implemented into a regular incoming goods department.

The invention may further be applied to a large number of authenticable medical products. As an example, generally, any type of product which may be shaped by injection molding may be named. Thus, many medical products, which generally may be counterfeited easily, consist of or comprise one or more injection-molded parts. As an example, lancets for puncture aids or disposable syringes for intravenously applied drugs may be named. In order to detect counterfeited lancets or syringes, one or more authentication bodies, such as one or more coated glass spheres, may be mixed into the master batch of the matrix material, such as one or more glass spheres having a known distribution of diameters and/or layer thicknesses. These coated spheres, generally, may be embodied such that these spheres, even in translucent plastic parts, are hardly visible with the naked eye. Still, by using imaging technology such as computer tomography, these spheres may easily be detected and measured, due to the higher x-ray density as compared to the surrounding plastic matrix material. Generally, a manufacturing process of the products and goods may be maintained and kept unchanged.

As a further example for potential authenticable medical products, test elements or test strips may be named, specifically test strips having at least one test chemical adapted for performing at least one detection reaction, specifically in case one or more analytes to be detected are present. The test chemical, which, as an example, realizes a specific detection within a sample of a body fluid, such as a blood sample, is typically applied to test strips, such as foil-shaped test strips. As an example, in the case of blood glucose measurements, these test strips are manufactured at a high volume. The test strips may be counterfeited rather easily, even though the test chemical itself is difficult to copy. A potential user may not be able to distinguish the counterfeited test strips, having a placebo test chemical thereon, from an original test strip having the original test chemical. Test strips, however, in many cases comprise a laminate of two or more layers. In order to implement the at least one authentication body into the test strip, without the authentication body being visible from the outside, the authentication body may simply be mixed into an adhesive which is used for laminating the layers of the test strip. The further steps of manufacturing the test strips may remain unchanged.

As a further example, pills or tablets for oral application may be named. These pills or tablets are often made from powders by application of pressure. In order to authenticate these products directly and independent from a primary packaging, one or more authentication bodies may be mixed into the powder and/or into a binder used for improving the integrity of the pills or tablets, such as by adding the authentication bodies as an additional filler material. The authentication body, as an example, may comprise a powder of coated particles, such as one or more of the above-mentioned particles. As an example, coated gold flakes may be used. Generally, gold is biocompatible and biologically inert, such that an ingression into the human body is generally harmless.

As a further example, complex components of a system may be used, such as puncture aids used for diabetes monitoring, e.g. composite lancets available under the trade name Accu-Chek® Softclix, available by Roche Diagnostics GmbH, Germany. The lancet of these Softclix systems generally comprises an injection-molded plastic part, with an integrated needle made of steel. The plastic body is relatively large and intransparent, and an introduction and detection of one or more authentication bodies is technically feasible.

As an example, in order to distinguish authentic lancets or syringes from falsifications, a master batch of matrix material may be mixed with glass spheres, such as glass spheres having a diameter of 0.2 mm and having a standard deviation of 0.02 mm. The glass spheres may be coated with a silver coating, such as a silver coating having a thickness of 50 microns and a standard deviation of 5 microns. The manufacturing of the product itself does not change. As an example, a master batch of thermoplastic material, such as ABS and/or POM, may be used, and the resulting mixture may be subject to an injection molding process in order to generate the above-mentioned lancets. The content of authentication bodies, such as glass spheres, in the injection-molded part, in this embodiment or other embodiments of the present invention, may be, as an example, 0.1 vol.-%. Other ranges are feasible.

As a further example, an infusion kit may be named. An infusion kit typically comprises one or more small tubes and one or more injection-molded parts, as well as one or more plasters and one or more needles. Typically, a fluid connector is standardized and, thus, may easily be counterfeited. Counterfeiting, however, may lead to severe quality problems, since, as an example, the tubing may break, which may lead to a dangerous situation for the patient. One or more application bodies may be introduced into the injection-molded parts and/or into the plasters, by using the above-mentioned techniques. When used in the plaster, due to the intransparency of the plaster, the one or more authentication bodies are generally invisible.

Further potential embodiments of the at least one authentication body refer to the use of one or more coated fibers as authentication body. As an example, glass fibers and/or steel fibers may be coated and used in the present invention. The sole prerequisite for using these fibers as authentication bodies is the detectability and distinguishability of these fibers in an appropriate imaging method, such as computer tomography, and a sufficient contrast to the surrounding matrix material. Further, the length and/or the diameter of these fibers may be rendered at low tolerance. Consequently, the length and/or the diameter of the core and/or the at least one cover layer of the fiber may be used as identification features for the purpose of authentication.

The authentication body may generally embodied as a nontoxic and/or biocompatible element, comprising non-allergenic or even anti-biotic substances, which may even be used for manufacturing food-safe or food-grade components, e.g. by using injection molding. As an example, suitable materials for authentication bodies are silicate glass and/or gold, e.g. gold flakes, coated with one or more cover layers.

Generally, the authentication body, as an example, may have the shape of a sphere, a fiber or flake. Other shapes are feasible, as outlined above. As a basic material, for instance, glass spheres, various types of fibers or flakes of foil may be used. As a material for forming the authentication body, as well as a material for the matrix material, thermoplastic materials, resins, rubbers, natural materials, glasses, metals, ceramic materials or other materials may be used. Further, technical materials, food-grade and/or medical-grade materials may be used. The authenticable medical product and/or parts thereof may be manufactured by using a wide variety of manufacturing processes, such as injection molding, extruding, casting, milling, forming or thermoforming, laminating, rapid prototyping or other manufacturing techniques. The products generally may have an arbitrary shape, such as a three-dimensional shape, or may be provided as a foil, a meadow or non-woven fabric, a fabric or a laminate.

The at least one authentication body, as outlined above, comprises one or more cover layers, wherein a single-layer or multi-layer setup may be used. The layer thickness or layer thicknesses of the cover layer or cover layers may be determined in the detection step, and/or a ratio of the layer thicknesses as well as comparison with a predetermined authentication criterion may be performed. Thus, for decoding or authentication, at least one parameter of the coated authentication body may be determined and compared with one or more predetermined values, such as a predetermined range of "allowed" thicknesses for authentication.

For manufacturing the coated authentication body, one or more layers of a resin or other coating materials may be applied to the core of the authentication body. For this purpose, various coating techniques may be used, such as a wet coating technique and/or evaporation techniques, such as physical vapor deposition and/or chemical vapor deposition.

The measured thickness, length or diameter of the application body, such as the spheres, fibers or flakes, may also be used for authentication.

The authentication, i.e. the decoding of the one or more authentication bodies, may also imply, as outlined above, a statistic distribution, such as a frequency of occurrence of certain layer thicknesses and/or diameters. These statistical distributions may be described in a graphical way and/or by deriving appropriate numbers, such as a mean value and/or a standard deviation. Further, instead of layer thicknesses, layer densities and/or the differences between layer densities may be used as a relevant criterion for authentication.

By using a dedicated sequence of a plurality of cover layers, each cover layer having an identifiable thickness and/or density, a code may be implemented, which may be changed e.g. on a regular basis, such as a code for encoding a batch number and/or other information. A batch code may also be implemented by a dedicated combination of e.g. spheres or fibers or of a combination of different types of coated authentication bodies.

For disguising authentication bodies in transparent products, transparent cores and transparent layers may be used. Further, decorative optical properties may be added, such as by implementing a metallic effect.

The detection and decoding of the authentication body may be performed in an automated fashion. For this purpose, the product may be scanned automatically and may be compared e.g. with a CAD model of the product. Therein, statistical distributions of one or more predetermined parameters may be generated. These statistical distributions may be compared with predetermined statistical distributions of the authentication body batches, in order to generate authentication information regarding the authenticity of the product.

As outlined above, the prior art generally describes embedding one or more homogeneous authentication bodies into a matrix material, wherein encoding is generally performed by using the spatial distribution of homogeneous authentication bodies. However, embedding heterogenic authentication bodies, such as one or more authentication bodies of a micron size, comprising a core material with one or more cover layers for encoding, as suggested by the present invention, provides a large number of advantages. Advantageously, such coding generally provides for hidden authentication, which is easy to incorporate in mass products and, at the same time, difficult to detect and copy. Particularly, using the layer thickness, the layer density or the thickness distribution of various layers, may provide a simple method for secure encoding and/or authentication. Simultaneously, the layer coding can also be used to generate further coding information, such as a batch or lot-coding for identification.

For disguising the authentication bodies, the matrix material, the layer material, the geometry (e.g. balls, plates or fibers) or the quantity of authentication bodies may also be adapted, depending on the application. For instance, the material of the authentication bodies may be adapted to be camouflaged and/or disguised via an optical effect, e.g. glitter particles in a transparent matrix material.

Preferably, these heterogenic authentication bodies are used in medical products, reaching from pharmaceuticals, in which the materials generally have to be biocompatible, and/or their packagings to medical devices. In this respect, medical products without biocompatibility issues, such as devices, test strips, packages, are of particular interest.

The above-mentioned detection of the authentication bodies including the decoding may easily be performed via computer tomography, in order to achieve the desired resolution and contrasts between different materials.

Summarizing the findings of the present invention, the following embodiments are preferred:
Embodiment 1: An authenticable medical product comprising a matrix material with at least one authentication body embedded into the matrix material, wherein the authentication body is heterogeneous and comprises:
   - a core material forming a core of the authentication body, and
   - at least one cover layer at least partially covering the core of the authentication body, wherein a material of the at least one cover layer is different from the core material and the matrix material.
Embodiment 2: The authenticable medical product according to the preceding embodiment, wherein the material of the at least one cover layer differs from the core material in such a way that the at least one cover layer is distinguishable from the core by using at least one detection method.
Embodiment 3: The authenticable medical product according to the preceding embodiment, wherein the detection method is a nondestructive imaging method.
Embodiment 4: The authenticable medical product according to the preceding embodiment, wherein the nondestructive imaging method comprises a tomographic imaging method.
Embodiment 5: The authenticable medical product according to any one of the two preceding embodiments, wherein the nondestructive imaging method comprises computer tomography and/or x-ray tomography.
Embodiment 6: The authenticable medical product according to any one of the preceding embodiments, wherein the material of the at least one cover layer differs from the core material by at least one property rendering the core and the cover layer distinguishable by x-ray imaging, preferably at least one property selected from the group consisting of an x-ray density or an x-ray absorption coefficient.
Embodiment 7: The authenticable medical product according to any one of the preceding embodiments, wherein the material of the at least one cover layer differs from the matrix material by at least one property rendering the matrix material and the cover layer distinguishable by x-ray imaging, preferably at least one property selected from the group consisting of an x-ray density or an x-ray absorption coefficient.
Embodiment 8: The authenticable medical product according to any one of the preceding embodiments, wherein the core material differs from the matrix material by at least one property rendering the core and the matrix material distinguishable by x-ray imaging, preferably at least one property selected from the group consisting of an x-ray density or an x-ray absorption coefficient.
Embodiment 9: The authenticable medical product according to any one of the preceding embodiments, wherein the core, the cover layer and the matrix material are distinguishable by x-ray imaging.
Embodiment 10: The authenticable medical product according to any one of the preceding embodiments, wherein a plurality of authentication bodies is provided in the matrix material.
Embodiment 11: The authenticable medical product according to any one of the preceding embodiments, wherein the authentication body has a diameter or equivalent diameter of 0.1 µm to 500 µm, such as 1 µm to 100 µm.
Embodiment 12: The authenticable medical product according to any one of the preceding embodiments, wherein the at least one authentication body comprises a plurality of particles.
Embodiment 13: The authenticable medical product according to any one of the preceding embodiments, wherein the matrix material is selected from the group consisting of: a plastic material; a glass material; a ceramic material; a metal; a varnish paint.
Embodiment 14: The authenticable medical product according to any one of the preceding embodiments, wherein the matrix material comprises a homogeneous matrix material.
Embodiment 15: The authenticable medical product according to any one of the preceding embodiments, wherein the matrix material comprises a plurality of matrix material layers, wherein the at least one authentication body is embedded in between at least two of the matrix material layers.
Embodiment 16: The authenticable medical product according to any one of the preceding embodiments, wherein the at least one authentication body is selected from the group consisting of: a sphere; a fiber; an amorphous particle; a lamella; a flake; a bead.
Embodiment 17: The authenticable medical product according to any one of the preceding embodiments, wherein the core material is selected from the group consisting of: a plastic material; a thermoplastic material; a resin; a rubber; a natural material; a ceramic material; a metal; a metal alloy; a glass; a quartz; a crystalline material; a silicone; a nanotube.
Embodiment 18: The authenticable medical product according to any one of the preceding embodiments, wherein the authentication body has a multilayer setup having a multiplicity of cover layers.
Embodiment 19: The authenticable medical product according to any one of the preceding embodiments, wherein the product comprises at least one additional disguise material embedded into the matrix material, wherein the disguise material lowers a detectability of the authentication body.
Embodiment 20: The authenticable medical product according to the preceding embodiment, wherein the disguise material comprises barium sulfate; zirconium oxide; bismuth vanadate; titanium dioxide.
Embodiment 21: The authenticable medical product according to any one of the preceding embodiments, wherein the authentication body is invisible from the outside under inspection by the naked eye.
Embodiment 22: The authenticable medical product according to any one of the preceding embodiments, wherein the authenticable medical product is selected from the group consisting of: a puncture aid for generating at least one opening in a skin of a user; a lancet; a syringe; an infusion device for administering a liquid to a body tissue; a plaster; a packaging for a medical device or product; a pharmaceutical; a test element for detecting at least one property of a sample; a test strip; an insertable sensor, specifically for continuous monitoring; a meter housing; an electronic circuit board; a sensor for continuous subcuteanous monitoring; a circuit board for an electronic medical device; a carrier with a plurality of test pads.
Embodiment 23: An authentication device for authentication of at least one authenticable medical product according to any one of the preceding embodiments, the authentication device comprising at least one detector for detecting the at least one authentication body and for distinguishing the core and the cover layer of the authentication body, the authentication device further comprising at least one evaluation device, the evaluation device being adapted for decoding the at least one authentication body.
Embodiment 24: The authentication device according to the preceding embodiment, wherein the detector comprises at least one tomographic imaging device.
Embodiment 25: The authentication device according to any one of the two preceding embodiments, wherein the evaluation device comprises at least one processor.
Embodiment 26: The authentication device according to any one of the three preceding embodiments, wherein the evaluation device is adapted to generate at least one item of information regarding an authenticity of the authenticable medical product.
Embodiment 27: An authentication system comprising the authentication device according to any one of the four preceding embodiments, the authentication system further comprising the authenticable medical product according to any one of the preceding embodiments referring to an authenticable medical product.
Embodiment 28: An authentication method for testing authenticity of the authenticable medical product according to any one of the preceding embodiments referring to an authenticable medical product, the authentication method comprising detecting and decoding the at least one authentication body embedded into the authenticable medical product.
Embodiment 29: The authentication method according to the preceding embodiment, wherein the detecting of the at least one authentication body comprises using at least one nondestructive imaging method.
Embodiment 30: The authentication method according to the preceding embodiment, wherein the nondestructive imaging method comprises a tomographic imaging method.
Embodiment 31: The authentication method according to any one of the two preceding embodiments, wherein the nondestructive imaging method comprises computer tomography and/or x-ray tomography.
Embodiment 32: The authentication method according to any one of the preceding method embodiments, wherein the detecting of the at least one authentication body comprises generating at least one three-dimensional image of at least a part of the authenticable medical product.
Embodiment 33: The authentication method according to any one of the preceding method embodiments, wherein the detecting of the at least one authentication body comprises detecting one or more of: a contrast between the matrix material and the core material; a contrast between the matrix material and the material of the at least one cover layer; a contrast between the core material and the material of the at least one cover layer.
Embodiment 34: The authentication method according to any one of the preceding method embodiments, wherein the decoding the at least one authentication body comprises deriving at least one measurement value from the detecting of the at least one authentication body.
Embodiment 35: The authentication method according to the preceding embodiment, wherein the decoding further comprises comparing the at least one measurement value with at least one nominal value or nominal value range.
Embodiment 36: The authentication method according to any one of the two preceding embodiments, wherein the measurement value comprises one or more of: a diameter or equivalent diameter of the authentication body; a geometric shape of the authentication body; a thickness of the cover layer; a mean diameter of a plurality of authentication bodies; a mean thickness of cover layers; a spatial distribution of authentication bodies; a geometric shape of the authentication body.
Embodiment 37: The authentication method according to any one of the preceding method embodiments, wherein the decoding the at least one authentication body comprises generating at least one authentication result.
Embodiment 38: The authentication method according to the preceding embodiment, wherein the authentication result comprises at least one item of information regarding an authenticity of the authenticable medical product.
Embodiment 39: The authentication method according to any one of the preceding method embodiments, wherein the decoding the at least one authentication body comprises generating at least one additional item of information encoded in the authenticable medical product.
Embodiment 40: The authentication method according to the preceding embodiment, wherein the at least one additional item of information comprises at least one item of information characterizing one or more of: an identity of the authenticable medical product; a manufacturer of the authenticable medical product; a vendor of the authenticable medical product; a distributor of the authenticable medical product; a property of the authenticable medical product; an intended use of the authenticable medical product; a calibration of the authenticable medical product; a batch number of the authenticable medical product.

### Short description of the Figures

Further optional features and embodiments of the invention will be disclosed in more detail in the subsequent description of preferred embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figure 1: shows an exemplary embodiment of an authenticable medical product;
- Figure 2: shows an exemplary embodiment of an authentication device and an authen-tication system; and
- Figures 3A and 3B: show various results of an authentication method, with a positive authentication (Figure 3A) and a negative authentication (Figure 3B).

### Detailed description of the embodiments

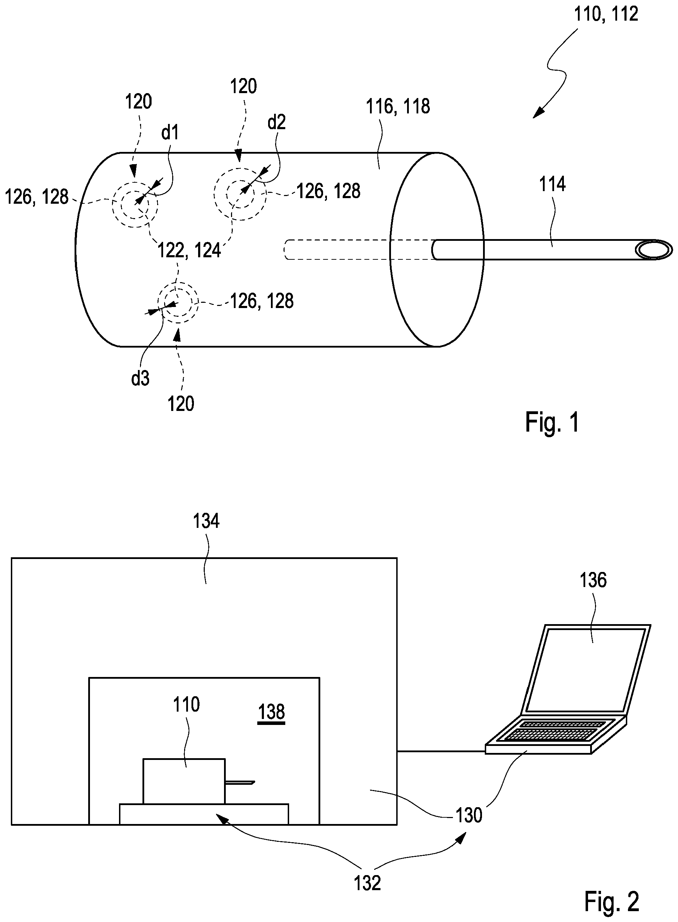

In Figure 1, an exemplary embodiment of an authenticable medical product 110 is disclosed in a schematic fashion. In this exemplary embodiment, the authenticable medical product comprises a puncture aid 112, having a lancet 114, made of a metal such as stainless steel, the lancet 114 is embedded into the body 116 made of a plastic material. As an example, the base body may be made of a thermoplastic material such as ABS or POM. The thermoplastic material, in this embodiment, forms a matrix material 118, into which, in this embodiment, a plurality of authentication bodies 120 are embedded, which, in this schematic figure, are depicted at an exaggerated size, for clarification purposes.

As an example, the authentication bodies 120 may comprise glass spheres, such as glass spheres having a nominal or mean diameter of 0.1 to 0.5 mm, e.g. 0.2 mm, with a predetermined standard deviation. As an example, the standard deviation may be 0.01 mm to 0.05 mm, such as 0.02 mm. The glass spheres form a core 122 of the authentication bodies, wherein the glass of the glass spheres forms a core material 124 of the core.

The glass spheres are coated with one or more cover layers 126, being formed of a cover layer material 128, also referred to as "the material of the cover layer". As an example, the cover layer material 128 may be silver. Each cover layer 126 has a thickness, in Figure 1 symbolically depicted by d1, d2, d3. As an example, the thickness d of the silver cover layer may be 5 µm to 100 µm, such as around 50 µm, e.g. with a standard deviation such as 5 µm.

In order to manufacture the authenticable medical product 110, as outlined above, the coated authentication bodies 120 may be mixed into a master batch of the matrix material 118, and the master batch may be used for an injection molding process forming the puncture aid 112. The content of the authentication bodies 120, by volume, may be, e.g., 0.01 to 5 %, such as 0.05 to 1 %, e.g. 0.1 % by volume. Other embodiments are feasible.

In Figure 2, a schematic setup of an authentication device 130 and an authentication system 132 is disclosed. The authentication device comprises at least one detector 134 which, as an example, may be embodied as a computer tomography device (CT). Further, the authentication device comprises at least one evaluation device 136, such as a computer and/or a processor, which is adapted for evaluating signals provided by the detector 134 and which is adapted for decoding information contained in the authentication bodies 120 of an authenticable medical product 110. The authenticable medical product 110 to be authenticated by the authentication device 130 as well as the authentication device 130 itself form the authentication system 132. As an example, the authenticable medical product 110 to be authenticated by the authentication device 130 may be located in a sample receptacle 138 of the detector 134. The authentication process may further be performed in an automated fashion, such as by providing one or more actuators which automatically transfer one authenticable medical product 110 after the other into the sample receptacle 138.

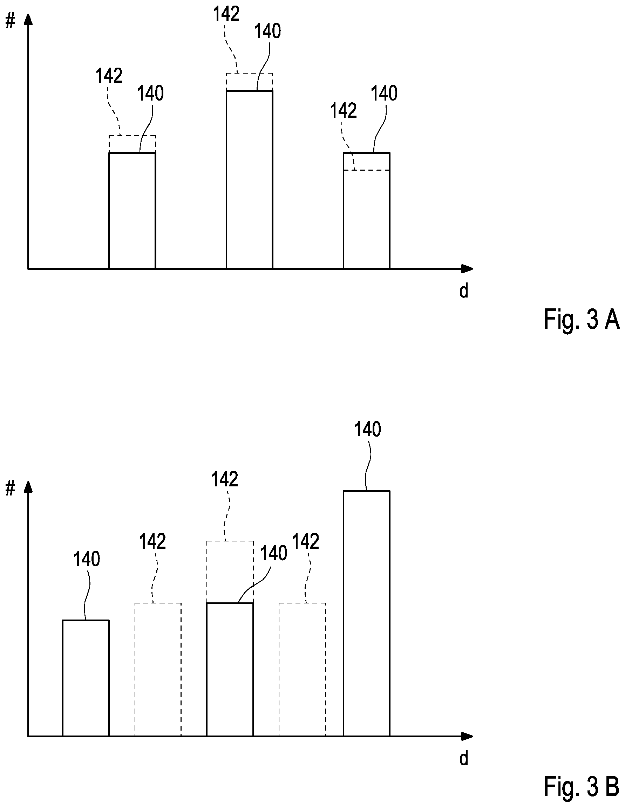

In Figures 3A and 3B, in an exemplary embodiment, an authentication method for testing authenticity of an authenticable medical product 110 is disclosed. By evaluating data provided by the detector 134 by using the evaluation device 136, as an example, a size distribution or thickness distribution of the thicknesses d of the cover layers 126 is generated. This is possible due to the fact that the matrix material 118, the core material 124 and the cover layer material 128 are distinguishable in images captured by the detector 134, such as the computer tomography device, e.g. due to their distinguishable x-ray absorption coefficient and/or x-ray density. In Figures 3A and 3B, in an exemplary fashion, histograms of the number of particles # as a function of the layer thickness d of the cover layers 126 are shown. Therein, the solid lines of the bars, denoted by reference number 140, render the actually measured distribution of the layer thicknesses, wherein, indicated by dashed lines and reference numbers 142, a predetermined distribution 142 is also given. The predetermined distribution 142 of layer thicknesses is compared with measured distribution 142, in order to authenticate the authenticable medical product 110. As depicted in Figure 3A, the product more or less matches the authentication criterion, since bars 140 and 142, at least to a predetermined or determinable degree of tolerance, match. Contrarily, in Figure 3B, the measured distribution 140 significantly deviates from the predetermined distribution 142. Consequently, the authentication method in Figure 3A renders a positive result, indicating that the authenticable medical product 110 is an authentic product, whereas in Figure 3B, the authentication method renders a negative result, indicating that the authenticable medical product 110 is not authentic.

The means and methods depicted in Figures 1 to 3 may be varied along the lines and within the scope of the present invention in various ways. Thus, as outlined above, other types of matrix materials 118 and/or other types of authentication bodies 120 may be used. Further, other authentication criteria may be applied, such as by using a multi-layer setup, with authentication information encoded in the multi-layer setup. Further, other authentication criteria may be used and may be detected by using the detector 134, such as a shape of the authentication bodies 120, other dimensions of the authentication bodies 120 such as a core diameter of the cores 122, x-ray densities or the like. Further, combinations of authentication criteria may be used. It shall be noted that, in addition to the simple authentication information "authentic" or "not authentic", additional information may be encoded by using the authentication bodies 120. Thus, as an example, batch numbers, lot numbers, vendor information, distributor information, information on an intended use, manufacturing dates, expiry dates or other information may be encoded by using the authentication bodies 120. Thus, as outlined above, a layer sequence of a multi-layer setup may be used to encode specific information. This additional information may also be read out by using the authentication device 130.

In the following, further non-limiting examples of authenticable medical products 110 are given.

### Additional example 1:

Authenticable medical products 110 in the form of capillary blood glucose test strips are manufactured as described e.g. in EP 1 039 298 B1, with the modification that the adhesive contains active carbon and additionally 0.1 % of a core-shell pigment. Therein, a core made of SiO₂ and a shell of ZnSiO₄ are used.

### Additional example 2:

An authenticable medical product 110 in the form of a Luer conus of an infusion set is manufactured by injection molding. A master batch of polypropylene containing 1 % (w/w) of a core-shell pigment is mixed to the polymer. Again, a core made of SiO₂ and a shell made of ZnSiO₄ are used.

### Additional example 3:

An authenticable medical product 110 in the form of a plastic body as guiding element for a lancet is manufactured by injection molding. A master batch of polypropylene containing 1 % (w/w) of a core-shell pigment and 5% BaSO₄ (as a disguise material) are mixed to the polymer. Again, a core made of SiO₂ and a shell made of ZnSiO₄ are used.

### Additional example 4:

An authenticable medical product 110 in the form of an analytical test tape is manufactured, as described in WO 2005/032372 A1, with white color fields between the reagent pads. These white fields are printed onto the carrier tape before application of the reagent pads. The ink contains a small amount (< 0,1 % w/w) of core-shell pigment particles in addition to a large amount of TiO₂.

### Additional example 5:

An authenticable medical product 110 in the form of a plastic body to be used as part of the housing for a sensor for subcutaneous continuous blood glucose monitoring (SCGM) is manufactured by injection molding. Therein, a master batch of polypropylene containing 1 % (w/w) of a core-shell pigment and 5% TiO₂ as a disguise material are mixed to the polymer. Again, a core made of SiO₂ and a shell made of ZnSiO₄ are used.

### Additional example 6:

An authenticable medical product 110 in the form of a circuit board of a blood glucose meter or a section thereof is manufactured. Onto a section of the circuit board, a lacquer coat is printed, which contains a small amount (< 0,1 % w/w) of core-shell pigment particles as authentication bodies 120.

For detecting and decoding the at least one authentication body 120 in the additional example 6 above, a two-step process may be applied: In a first step, an infrared image of the printed region may be taken for determination of a "region-of-interest". This region of interest, in a second step, may then be analyzed, such as by nano-tomography. Core diameter and shell thickness of the authentication bodies 120 may be decoded for retrieving information (e.g. year of manufacture) encoded therein.

In the additional examples disclosed above as well as in other examples, specifically, the core-shell pigment preparations may have a core diameter (e.g. a mean diameter or average diameter) of e.g. either 5 µm or 10 µm and a shell thickness (such as a mean or average thickness) of 50 nm or 100 nm. Thereof, a plurality of different combinations may be formed and may be used for encoding the information.

### List of reference numbers

- 110: authenticable medical product
- 112: puncture aid
- 114: lancet
- 116: bare body
- 118: matrix material
- 120: authentication body
- 122: core
- 124: core material
- 126: cover layer
- 128: cover layer material
- 130: authentication device
- 132: authentication system
- 134: detector
- 136: evaluation device
- 138: sample receptacle
- 140: measured distribution
- 142: predetermined distribution

## Claims

1. An authenticable medical product (110) comprising a matrix material (118) with at least one authentication body (120) embedded into the matrix material (118), wherein the authentication body (120) is heterogeneous and comprises:
- a core material (124) forming a core (122) of the authentication body (120), and
- at least one cover layer (126) at least partially covering the core (122) of the authentication body (120), wherein a material (128) of the at least one cover layer (126) is different from the core material (124) and the matrix material (118).

2. The authenticable medical product (110) according to the preceding claim, wherein the material (128) of the at least one cover layer (126) differs from the core material (124) in such a way that the at least one cover layer (126) is distinguishable from the core (122) by using at least one detection method, wherein the detection method is a nondestructive imaging method, wherein the nondestructive imaging method comprises computer tomography and/or x-ray tomography.

3. The authenticable medical product (110) according to any one of the preceding claims, wherein the core (122), the cover layer (126) and the matrix material (118) are distinguishable by x-ray imaging.

4. The authenticable medical product (110) according to any one of the preceding claims, wherein the authentication body (120) has a diameter or equivalent diameter of 0.1 µm to 500 µm.

5. The authenticable medical product (110) according to any one of the preceding claims, wherein the authentication body (120) has a multilayer setup having a multiplicity of cover layers (126).

6. The authenticable medical product (110) according to any one of the preceding claims, wherein the authenticable medical product (110) further comprises at least one additional disguise material embedded into the matrix material (118), wherein the disguise material lowers a detectability of the authentication body (120).

7. The authenticable medical product (110) according to any one of the preceding claims, wherein the authenticable medical product (110) is selected from the group consisting of: a puncture aid (112) for generating at least one opening in a skin of a user; a lancet; a syringe; an infusion device for administering a liquid to a body tissue; a plaster; a packaging for a medical device or product; a pharmaceutical; a test element for detecting at least one property of a sample; a test strip; an insertable sensor; a meter housing; an electronic circuit board; a sensor for continuous subcuteanous monitoring; a circuit board for an electronic medical device; a carrier with a plurality of test pads.

8. An authentication device (130) for authentication of at least one authenticable medical product (110) according to any one of the preceding claims, the authentication device (130) comprising at least one detector (134) for detecting the at least one authentication body (120) and for distinguishing the core (122) and the cover layer (126) of the authentication body (120), the authentication device (130) further comprising at least one evaluation device (136), the evaluation device (136) being adapted for decoding the at least one authentication body (120).

9. An authentication system (132), comprising the authentication device (130) according to the preceding claim, the authentication system (132) further comprising the authenticable medical product (110) according to any one of the preceding claims referring to an authenticable medical product (110).

10. An authentication method for testing authenticity of the authenticable medical product (110) according to any one of the preceding claims referring to an authenticable medical product (110), the authentication method comprising detecting and decoding the at least one authentication body (120) embedded into the authenticable medical product (110).

11. The authentication method according to the preceding claim, wherein the detecting of the at least one authentication body (120) comprises using at least one nondestructive imaging method, wherein the nondestructive imaging method comprises computer tomography and/or x-ray tomography.

12. The authentication method according to any one of the preceding method claims, wherein the decoding the at least one authentication body (120) comprises deriving at least one measurement value from the detecting of the at least one authentication body (120).

13. The authentication method according to the preceding claim, wherein the decoding further comprises comparing the at least one measurement value with at least one nominal value or nominal value range.

14. The authentication method according to any one of the preceding method claims, wherein the decoding the at least one authentication body (120) comprises generating at least one authentication result, wherein the authentication result comprises at least one item of information regarding an authenticity of the authenticable medical product (110).

15. The authentication method according to any one of the preceding method claims, wherein the decoding the at least one authentication body (120) comprises generating at least one additional item of information encoded in the authenticable medical product (110).

## Patentansprüche

1. Authentifizierbares Medizinprodukt (110), umfassend ein Matrixmaterial (118) mit mindestens einem in dem Matrixmaterial (118) eingebetteten Authentifizierungskörper (120), wobei der Authentifizierungskörper (120) heterogen ist und Folgendes umfasst:
- ein Kernmaterial (124), das einen Kern (122) des Authentifizierungskörpers (120) bildet und
- mindestens eine Abdeckschicht (126), die den Kern (122) des Authentifizierungskörpers (120) zumindest teilweise abdeckt, wobei sich ein Material (128) der mindestens einen Abdeckschicht (126) vom Kernmaterial (124) und vom Matrixmaterial (118) unterscheidet.

2. Authentifizierbares Medizinprodukt (110) nach dem vorhergehenden Anspruch, wobei sich das Material (128) der mindestens einen Abdeckschicht (126) vom Kernmaterial (124) derart unterscheidet, dass die mindestens eine Abdeckschicht (126) vom Kern (122) unter Verwendung mindestens eines Nachweisverfahrens unterscheidbar ist, wobei das Nachweisverfahren ein nicht-destruktives Bildgebungsverfahren ist, wobei das nicht-destruktive Bildgebungsverfahren Computertomographie und/oder Röntgentomographie umfasst.

3. Authentifizierbares Medizinprodukt (110) nach einem der vorhergehenden Ansprüche, wobei der Kern (122), die Abdeckschicht (126) und das Matrixmaterial (118) durch Röntgenbildgebung voneinander unterscheidbar sind.

4. Authentifizierbares Medizinprodukt (110) nach einem der vorhergehenden Ansprüche, wobei der Authentifizierungskörper (120) einen Durchmesser oder äquivalenten Durchmesser von 0,1 µm bis 500 µm aufweist.

5. Authentifizierbares Medizinprodukt (110) nach einem der vorhergehenden Ansprüche, wobei der Authentifizierungskörper (120) einen mehrschichtigen Aufbau mit einer Vielzahl von Abdeckschichten (126) aufweist.

6. Authentifizierbares Medizinprodukt (110) nach einem der vorhergehenden Ansprüche, wobei das authentifizierbare Medizinprodukt (110) ferner mindestens ein zusätzliches, im Matrixmaterial (118) eingebettetes Maskierungsmaterial aufweist, wobei das Maskierungsmaterial eine Nachweisbarkeit des Authentifizierungskörpers (120) verringert.

7. Authentifizierbares Medizinprodukt (110) nach einem der vorhergehenden Ansprüche, wobei das authentifizierbare Medizinprodukt (110) aus der folgenden Gruppe ausgewählt ist: eine Punktionshilfe (112) zur Erzeugung mindestens einer Öffnung in einer Haut eines Benutzers; eine Lanzette; eine Spritze; eine Infusionsvorrichtung zur Verabreichung einer Flüssigkeit an ein Körpergewebe; ein Pflaster; eine Verpackung für eine medizinische Vorrichtung oder ein Medizinprodukt; ein pharmazeutisches Mittel; ein Testelement für den Nachweis mindestens einer Eigenschaft einer Probe; ein Teststreifen; ein einführbarer Sensor; ein Messgerätgehäuse; eine elektronische Leiterplatte; ein Sensor zur kontinuierlichen subkutanen Überwachung; eine Leiterplatte für eine elektronische medizinische Vorrichtung; ein Träger mit einer Vielzahl von Testfeldern.

8. Authentifizierungsvorrichtung (130) zur Authentifizierung mindestens eines authentifizierbaren Medizinprodukts (110) nach einem der vorhergehenden Ansprüche, wobei die Authentifizierungsvorrichtung (130) mindestens einen Detektor (134) zum Nachweisen des mindestens einen Authentifizierungskörpers (120) und zum Unterscheiden des Kerns (122) und der Abdeckschicht (126) des Authentifizierungskörpers (120) umfasst, wobei die Authentifizierungsvorrichtung (130) ferner mindestens eine Evaluierungsvorrichtung (136) umfasst, wobei die Evaluierungsvorrichtung (136) zum Entschlüsseln des mindestens einen Authentifizierungskörpers (120) ausgelegt ist.

9. Authentifizierungssystem (132), umfassend die Authentifizierungsvorrichtung (130) nach dem vorhergehenden Anspruch, wobei das Authentifizierungssystem (132) ferner das authentifizierbare Medizinprodukt (110) nach einem der vorhergehenden Ansprüche, die sich auf ein authentifizierbares Medizinprodukt (110) beziehen, umfasst.

10. Authentifizierungsverfahren zur Überprüfung der Authentizität des authentifizierbaren Medizinprodukts (110) nach einem der vorhergehenden Ansprüche, die sich auf ein authentifizierbares Medizinprodukt (110) beziehen, wobei das Authentifizierungsverfahren den Nachweis und die Entschlüsselung des mindestens einen, in dem authentifizierbare Medizinprodukt (110) eingebetteten Authentifizierungskörpers (120) umfasst.

11. Authentifizierungsverfahren nach dem vorhergehenden Anspruch, wobei der Nachweis des mindestens einen Authentifizierungskörpers (120) die Verwendung mindestens eines nicht-destruktiven Bildgebungsverfahrens umfasst, wobei das nicht-destruktive Bildgebungsverfahren Computertomographie und/oder Röntgentomographie umfasst.

12. Authentifizierungsverfahren nach einem der vorhergehenden Verfahrensansprüche, wobei die Entschlüsselung des mindestens einen Authentifizierungskörpers (120) die Ableitung mindestens eines Messwerts aus dem Nachweis des mindestens einen Authentifizierungskörpers (120) umfasst.

13. Authentifizierungsverfahren nach dem vorhergehenden Anspruch, wobei die Entschlüsselung ferner den Vergleich des mindestens einen Messwerts mit mindestens einem Nennwert oder einem Nennwertbereich umfasst.

14. Authentifizierungsverfahren nach einem der vorhergehenden Verfahrensansprüche, wobei die Entschlüsselung des mindestens einen Authentifizierungskörpers (120) das Generieren mindestens eines Authentifizierungsresultats umfasst, wobei das Authentifizierungsresultat mindestens eine Information bezüglich der Authentizität des authentifizierbaren Medizinprodukts (110) umfasst.

15. Authentifizierungsverfahren nach einem der vorhergehenden Verfahrensansprüche, wobei die Entschlüsselung des mindestens einen Authentifizierungskörpers (120) das Generieren mindestens einer zusätzlichen Information, die im authentifizierbaren Medizinprodukt (110) kodiert ist, umfasst.

## Revendications

1. Produit médical authentifiable (110) comprenant un matériau de matrice (118) avec au moins un corps d'authentification (120) incorporé dans le matériau de matrice (118), dans lequel le corps d'authentification (120) est hétérogène et comprend :
- un matériau de noyau (124) formant un noyau (122) du corps d'authentification (120), et
- au moins une couche de couverture (126) couvrant au moins partiellement le noyau (122) du corps d'authentification (120), dans lequel un matériau (128) de l'au moins une couche de couverture (126) est différent du matériau de noyau (124) et du matériau de matrice (118).

2. Produit médical authentifiable (110) selon la revendication précédente, dans lequel le matériau (128) de l'au moins une couche de couverture (126) diffère du matériau de noyau (124) de manière à ce que l'au moins une couche de couverture (126) puisse être distinguée du noyau (122) en utilisant au moins un procédé de détection, dans lequel le procédé de détection est un procédé d'imagerie non destructif, dans lequel le procédé d'imagerie non destructif comprend la tomodensitométrie et/ou la tomographie aux rayons X.

3. Produit médical authentifiable (110) selon l'une quelconque des revendications précédentes, dans lequel le noyau (122), la couche de couverture (126) et le matériau de matrice (118) peuvent être distingués par imagerie aux rayons X.

4. Produit médical authentifiable (110) selon l'une quelconque des revendications précédentes, dans lequel le corps d'authentification (120) a un diamètre ou diamètre équivalent de 0,1 µm à 500 µm.

5. Produit médical authentifiable (110) selon l'une quelconque des revendications précédentes, dans lequel le corps d'authentification (120) a une installation multicouche ayant une multiplicité de couches de couverture (126).

6. Produit médical authentifiable (110) selon l'une quelconque des revendications précédentes, dans lequel le produit médical authentifiable (110) comprend en outre au moins un matériau de camouflage supplémentaire incorporé dans le matériau de matrice (118), dans lequel le matériau de camouflage réduit une détectabilité du corps d'authentification (120).

7. Produit médical authentifiable (110) selon l'une quelconque des revendications précédentes, dans lequel le produit médical authentifiable (110) est choisi dans le groupe consistant en : une aide à la perforation (112) pour générer au moins une ouverture dans une peau d'un utilisateur ; un bistouri ; une seringue ; un dispositif d'infusion pour administrer un liquide à un tissu corporel ; un pansement ; un emballage pour un dispositif ou produit médical ; un produit pharmaceutique ; un élément d'essai pour détecter au moins une propriété d'un échantillon ; une bandelette réactive ; un capteur insérable ; un boîtier de compteur ; une carte de circuit électronique ; un capteur pour une surveillance sous-cutanée continue ; une carte de circuit pour un dispositif médical électronique ; un support avec une pluralité de tampons d'essai.

8. Dispositif d'authentification (130) pour l'authentification d'au moins un produit médical authentifiable (110) selon l'une quelconque des revendications précédentes, le dispositif d'authentification (130) comprenant au moins un détecteur (134) pour détecter l'au moins un corps d'authentification (120) et pour distinguer le noyau (122) et la couche de couverture (126) du corps d'authentification (120), le dispositif d'authentification (130) comprenant en outre au moins un dispositif d'évaluation (136), le dispositif d'évaluation (136) étant adapté pour décoder l'au moins un corps d'authentification (120).

9. Système d'authentification (132), comprenant le dispositif d'authentification (130) selon la revendication précédente, le système d'authentification (132) comprenant en outre le produit médical authentifiable (110) selon l'une quelconque des revendications précédentes se référant à un produit médical authentifiable (110).

10. Procédé d'authentification pour évaluer l'authenticité du produit médical authentifiable (110) selon l'une quelconque des revendications précédentes se référant à un produit médical authentifiable (110), le procédé d'authentification comprenant la détection et le décodage de l'au moins un corps d'authentification (120) incorporé dans le produit médical authentifiable (110).

11. Procédé d'authentification selon la revendication précédente, dans lequel la détection de l'au moins un corps d'authentification (120) comprend l'utilisation d'au moins un procédé d'imagerie non destructif, dans lequel le procédé d'imagerie non destructif comprend la tomodensitométrie et/ou la tomographie aux rayons X.

12. Procédé d'authentification selon l'une quelconque des revendications de procédé précédentes, dans lequel le décodage de l'au moins un corps d'authentification (120) comprend la dérivation d'au moins une valeur de mesure de la détection de l'au moins un corps d'authentification (120).

13. Procédé d'authentification selon la revendication précédente, dans lequel le décodage comprend en outre la comparaison de l'au moins une valeur de mesure avec au moins une valeur nominale ou plage de valeurs nominales.

14. Procédé d'authentification selon l'une quelconque des revendications de procédé précédentes, dans lequel le décodage de l'au moins un corps d'authentification (120) comprend la génération d'au moins un résultat d'authentification, dans lequel le résultat d'authentification comprend au moins une information concernant une authenticité du produit médical authentifiable (110).

15. Procédé d'authentification selon l'une quelconque des revendications de procédé précédentes, dans lequel le décodage de l'au moins un corps d'authentification (120) comprend la génération d'au moins une information supplémentaire codée dans le produit médical authentifiable (110).
